# EUROPEAN PATENT APPLICATION

(11) **EP 1 759 684 A1**
(43) Date of publication of application: **07.03.2007**
(21) Application number: 05019073.5
(22) Date of filing: 02.09.2005
(51) Int. Cl.: A61K 8/23

(54) **Process for bleaching keratin fibers**

(71) Applicant: Wella Aktiengesellschaft, 64274 Darmstadt (DE)
(72) Inventor: Javet, Manuela, 1723 Marly (CH); Müller, Catherine, 1723 Marly (CH); Weber, Ingo, 1723 Marly (CH)

(57) **Abstract**

The present patent application relates to a process for bleaching keratin fibers which is characterized by applying to the fiber a ready-to-use agent having a basic pH and containing
a) at least one bleach booster according to the general formula (I) 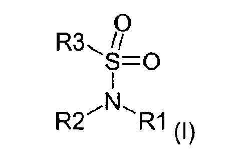 wherein **R1** is a hydrogen, **R2** is hydrogen, a substituted or unsubstituted C1- to C12-alkyl group, a substituted or unsubstituted C1- to C12-monohydroxy-alkyl group, a substituted or unsubstituted C2- to C12-polyhydroxy alkyl group, or a substituted saturated, unsaturated or aromatic 4- to 8-membered carbocycle or heterocycle, and **R3** is a OR-Group, with **R** being equal to hydrogen, an ammonium group or an alkali metal or an earth alkali metal atom; and
b) at least one appropriate type-2 bleach-stable direct dye and
c) at least one oxidant; and

after an exposure time of 5 to 60 minutes at a temperature of 10 to 70 °C rinsing the fiber with water;
as well as the use of special type-2 bleach-stable direct dyes for acquiring a natural looking bleaching of keratin fibers, particularly human hair, without undesired undertones.

## Description

The present invention relates to a process for bleaching keratin fibers in a pleasant and natural look by using bleaching agents which comprise special bleach boosters and direct dyes being stable against type-2 bleaches.

Natural hair color is derived from melanin granules embedded throughout the cortex of hair fibers. Two general classes of such pigments have been identified: eumelanins (brownish black) and pheomelanins (reddish orange). The combination ratio and concentration of these two types of pigments impart to the hair its characteristic natural gradations of color. Dark hair has a higher concentration of the eumelanins, while red hair has a predominance of the pheomelanins. Light blond hair has reduced amounts of both.

Human hair is arbitrarily assigned a scale often levels to describe its darkness (or lightness). Black hair is designated as level one, medium brown hair as level five, and pale light blond as level ten, with several nuances in-between.

Hair bleaching is a chemical process by which the melanin pigment granules are gradually destroyed by the bleaching agent, resulting in lighter hair color. The melanin pigments are not all lightened at the same rate. The eumelanins are easier to break down than the pheomelanins. Because of this property, dark hair, when bleached, experiences preferential destruction of the melanin pigments, which leads to the visual enhancement of the red pigments, and the casting of an undesirable warm reddish orange or "brassy" tone to the bleached hair. In order to neutralize this warmth, hair colorants of a drabbing nature are almost always applied during or after a bleaching treatment.

Based on their chemical composition and their strength, hair bleaches may be classified into two categories:
1. Type-1 bleaches: These, generally, are mild liquid- or creme-based compositions utilizing alkaline hydrogen peroxide solutions as the main oxygen-generating agent to oxidize and bleach the melanin, usually in conjunction with a hair coloring process. Just before use, the peroxide is mixed with an alkalizing agent such as ammonia, and the resulting liquid or creme is applied to hair for 30 to 60 minutes. Such compositions may lighten the hair by as much as 4 levels at the most, depending on the concentration of hydrogen peroxide used. For example, a level-6 hair may be lightened, under favorable conditions, to a level 10.
2. Type-2 bleaches: These are mostly powder compositions based on persulfate salts (ammonium, potassium, sodium) as auxiliary or booster supplies of active oxygen, and silicate and/or carbonate salts as sources of alkalinity. Again, just before use, they are mixed with hydrogen peroxide solutions to form a workable creme that can be applied to the hair. Some powder bleaches even have the hydrogen peroxide itself incorporated in a solid form such as urea peroxide. Quite often, a third separately-packaged component, referred to as a bleach oil which may contain humectants and other conditioning agents, is added to the bleach powder and peroxide at time of use.

Type-2 bleaches can deliver over seven levels of lift, something which cannot be attained with category-1 bleaches. They are usually utilized whenever more than four levels of lift are desired, such as when lifting a level-5 hair, or darker, to a pale blond.

Because of the underlying warm tones that are exposed at various levels of bleaching, hair lightening, as mentioned above, is generally accompanied by a toning process to neutralize the warmth and give the hair a pleasant natural look. The toning process itself is a rather delicate one. Toners fall into three hues: blue, green and violet, generally known as drabbing or ashing hues. These hues, or combinations of, are required to neutralize the spectrum of undertones that are exposed during the lightening process. Dark blond hair, for example, would expose yellow undertones upon bleaching. Therefore, according to the law of color, a violet-based toner would neutralize the yellowish tone to result in a platinum or silver blond shade. The concentration of the toner should be adjusted so that the lift is not masked by the deposition of color. Similarly, medium brown hair would reveal a significant amount of orange undertones, requiting a significant amount of a blue-based toner. Dark hair, when bleached, exhibits reddish-orange undertones requiring a bluish-green toner.

The type-1 bleaches constitute most of what is known as the current high-lifting shades of commercial permanent hair colorants. These colorants are often based on oxidative primary intermediates and couplers, and can contain direct dyes, disperse dyes, acid dyes, or basic dyes. The used alkaline peroxide environment is mild enough to allow the survival of several types of dyes.

In the type-2 bleaches, the medium is quite hostile to most dyes. The higher alkalinity and stronger oxidizing conditions act synergistically to destroy the present dyes within a short period of time. Therefore only few dyes can be used in combination with these bleaches.

There are many publications and patent applications or patents dealing with the art of simultaneous bleaching and coloring of hair, for example K. Schrader "Grundlagen und Rezepturen der Kosmetika", 2^{nd} edition (1989), page 783 ff, as well as US 5688291 A, US 3578387 A, US 3912446 A, DE 19961273 U1 and WO 97/39727 A1.

Normally, a type-1 bleach only allows a relatively small variation of the hair color because of the remaining undestroyed melanin. In addition common hair dyes don't survive the type-2 bleach process. In addition, type-2 bleaches show a lot of fiber damage and often a undesirable orange hue.

Surprisingly, we found bleach boosters which allow in combination with special typ-2 bleach-stable direct dyes to obtain bleachings with more natural and less orange hue in lighter shades. The fiber damage is comparable to a type-1 bleach.

It is known from DE 19961273 A1 to use special sulfamates as bleach booster.

The object of the present invention is a process for bleaching keratin fibers which is characterized by applying to the fiber a ready-to-use agent having a basic pH and containing
a) at least one bleach booster according to the general formula (I) 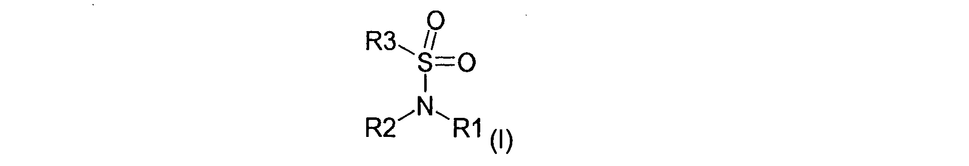 wherein **R1** is a hydrogen, **R2** is hydrogen, a substituted or unsubstituted C1- to C12-alkyl group, a substituted or unsubstituted C1- to C12-monohydroxy-alkyl group, a substituted or unsubstituted C2- to C12-polyhydroxy alkyl group, or a substituted saturated, unsaturated or aromatic 4- to 8-membered carbocycle or heterocycle, and **R3** is a OR-Group, with **R** being equal to hydrogen, an ammonium group or an alkali metal or an earth alkali metal atom; and
b) at least one appropriate type-2 bleach-stable direct dye and
c) at least one oxidant; and
   after an exposure time of 5 to 60 minutes at a temperature of 10 to 70 °C rinsing the fiber with water.

The bleach booster of the general formula (I) is preferably selected from among sodium cyclohexanesulfamate; ammonium cyclohexanesulfamate; potassium cyclohexanesulfamate; cyclohexane sulfamic acid (cyclamate); sulfamic acid; sodium sulfamate; potassium sulfamate;
ammonium sulfamate; sodium phenylsulfamate;
ammonium phenylsulfamate; potassium phenylsulfamate;
phenyl sulfamic acid; sodium benzylsulfamate;
ammonium benzylsulfamate; potassium benzylsulfamate;
benzyl sulfamic acid; sodium isopropylsulfamate;
ammonium isopropylsulfamate; potassium isopropylsulfamate;
isopropyl sulfamic acid; sodium methylsulfamate;
ammonium methylsulfamate; potassium methylsulfamate;
methyl sulfamic acid; sodium t-butylsulfamate;
ammonium t-butylsulfamate; potassium t-butylsulfamate and
t-butyl sulfamic acid.

The bleach booster is contained in the ready-to-use bleaching agent in an amount from about 0.01 to 70 weight percent and preferably from about 1 to 50 weight percent.

The type-2 bleach-stable direct dye is preferably selected from among 3-(2',6'-diaminopyridyl-3'-azo)pyridine (= 2,6-diamino-3-((pyridin-3-yl)azo)pyridine), N,N-di(2-hydroxyethyl)-3-methyl-4-((4-nitrophenyl)azo)-aniline (Disperse Red 17, CI11210), 3-diethylamino-7-(4-dimethylaminophenylazo)-5-phenylphenazinium chloride (CI11050), 4-(2-thiazolylazo)-resorcinol, 4-((4-phenylamino)azo)benzosulfonic acid sodium salt (Orange IV), 1-((3-aminopropyl)amino)-9,10-anthracenedione (HC Red No. 8), 3',3",4,5,5',5",6,7-octabromphenolsulfonphtalein (tetrabromophenol Blue), 1-((4-amino-3,5-dimethylphenyl)(2,6-dichlorophenyl)methylene)-3,5-dimethyl-4-imino-2,5-cyclohexadiene-phosphoric acid (1:1) (Basic Blue 77), 3',3",5',5"-tetrabromo-m-cresol sulfonephthalein, 2,4-dinitro-1-naphthol-7-sulfonic acid disodium salt (Acid Yellow 1, CI 10316), 4-[2'-hydroxy-1'-naphthyl)azo]benzosulfonic acid sodium salt (Acid Orange 7, CI15510), 3',6'-dihydroxy-2',4',5',7'-tetraiodospiro-[isobenzofuran-1(3H), 9'-(9H)xanthen]-3-one disodium salt (Acid Red 51, CI45430), 6-hydroxy-5-((2-methoxy-5-methyl-4-sulfophenyl)azo)-2-naphthalenesulfonic acid disodium salt (FD&C Red 40, CI16035), 2,4-dinitro-1-naphthol sodium salt (Acid Yellow 24; CI10315), 2',4',5',7'-tetrabromo-4,5,6,7-tetrachloro-3',6'-dihydroxyspiro-(isobenzofuran-1(3H), 9'-[9H]xanthen]-3-one disodium salt (Acid Red 92; CI45410), 4-(2-hydroxy-1-naphthylazo)-3-methylbenzenesulfonic acid sodium salt (Acid Orange 8, CI15575), 2-amino-1,4-naphthalenedione, dithizone (1,5-diphenylthiocarbazone), N-((2-hydroxyethyl)-2-nitro-4-trifluoromethyl)aniline (HC Yellow 13), N-(2-hydroxyethyl)-4-nitroaniline and 4-chloro-N-(2,3-dihydroxypropyl)-2-nitroaniline.

Particularly preferred type-2 bleach-stable direct dyes are N,N-di(2-hydroxyethyl)-3-methyl-4-((4-nitrophenyl)azo)-aniline (Disperse Red 17, Cl11210), 3-diethylamino-7-(4-dimethylamino-phenylazo)-5-phenylphenazinium chloride (CI11050), 4-(2-thiazolylazo)-resorcinol, 1-((3-aminopropyl)amino)-9,10-anthracenedione (HC Red No. 8), 3',3",4,5,5',5",6,7-octabromophenolsulfonphtalein (tetrabromophenol Blue), 1-((4-amino-3,5-dimethylphenyl)(2,6-dichlorophenyl)methylene)-3,5-dimethyl-4-imino-2,5-cyclohexadiene-phosphoric acid (1:1) (Basic Blue 77), 3',3",5',5"-tetrabromo-m-cresol sulfonephthalein, 3',6'-dihydroxy-2',4',5',7'-tetraiodospiro-[isobenzofuran-1(3H), 9'-(9H)xanthen]-3-one disodium salt (Acid Red 51, CI45430), 6-hydroxy-5-((2-methoxy-5-methyl-4-sulfophenyl)azo)-2-naphthalenesulfonic acid disodium salt (FD&C Red 40, Cl16035), 2',4',5',7'-tetrabromo-4,5,6,7-tetrachloro-3',6'-dihydroxyspiro-(isobenzofuran-1(3H), 9'-[9H]xanthen]-3-one disodium salt (Acid Red 92; CI45410), 2-amino-1,4-naphthalenedione and dithizone (1,5-diphenylthiocarbazone).

The direct dyes are contained in the ready-to-use agent in a total amount from about 0.0001 to 10 weight percent, preferably from about 0.001 to 5 weight percent, and most preferably from about 0.005 to 2 weight percent.

The agents according to the present invention also comprise at least one source of an oxidizing agent. Preferred oxidizing agents for use herein are water-soluble peroxygen oxidizing agents. "Water-soluble" as defined herein means that in standard condition at least 0.1 g, preferably 1 g, more preferably 10 g of said oxidizing agent can be dissolved in 1 liter of deionized water. The oxidizing agents are valuable for the initial solubilization and decolorization of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulfates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. The oxidizing agents may be provided in aqueous solution or as a powder which is dissolved prior to use. Preferred for use in the agents according to the present invention are hydrogen peroxide, percarbonates, persulfates and combinations thereof.

According to the present invention the agents comprise from about 0.1 % to about 15% by weight, preferably from about 1% to about 10% by weight, and most preferably from about 2% to about 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 10.5, preferably 7.5 to 10.5 more preferably about pH 10. Moreover, this system is also particularly effective in combination with a source of ammonia or ammonium ions. It has been found that this oxidizing agent can deliver improvements to the desired hair color results particularly with regard to the delivery of high lift, whilst considerably reducing the odor, skin and scalp irritation and damage to the hair fibers.

Accordingly, any source of these ions may be utilized. Suitable sources for use herein include sodium, potassium, guanidine, arginine, lithium, calcium, magnesium, barium, ammonium salts of carbonate, carbamate and hydrogencarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the agents may contain from about 0.1 % to about 15% by weight, preferably from about 1% to about 10% by weight, and most preferably from about 1 % to about 8% by weight of a hydrogencarbonate ion and from about 0.1 % to about 10% by weight, preferably from about 1 % to about 7% by weight, and most preferably from about 2% to about 5% by weight of a source of hydrogen peroxide.

According to the present invention the agent may further optionally comprise at least one source of alkalizing agent, preferably a source of ammonium ions and or ammonia. Any agent known in the art may be used such as alkanolamides for example monoethanolamine, diethanolamine, triethanolamine, monopropanolamine, dipropanolamine, tripropanolamine, 2-amino-2-methyl-1, 3-propanediol, 2-amino-2-methyl-1-propanol, and 2-amino-2-hydroxymethyl-1,3-propanediol and guanidium salts. Particularly preferred alkalizing agents are those which provide a source of ammonium ions. Any source of ammonium ions is suitable for use herein.

Preferred sources include ammonium chloride, ammonium sulfate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium carbamate, ammonium hydrogen carbonate, ammonia and mixtures thereof. The agents of the present invention may comprise from about 0.1 % to about 10% by weight, preferably from about 0.5% to about 5%, most preferably from about 1 % to about 3% of an alkalizing agent, preferably ammonium ions.

The agent according to the invention may also contain one or more additive commonly used in solutions, creams, emulsions, gels or aerosol foams, powders and granulates, for example solvents, such as water, low aliphatic monohydroxy or polyhydroxy alcohols, their esters and ethers, for example alkanols, particularly with an alkyl chain comprising 1 to 4 carbon atoms, such as ethanol, n-propanol or isopropanol, butanol, isobutanol; bivalent or trivalent alcohols, particularly of the type having 2 to 6 carbon atoms, such as ethylene glycol, propylene glycol, 1,3-propanediol, 1,4-butanediol, 1,5-pentanediol, 1,6-hexanediol, 1,2,6-hexanetriol, glycerine, diethylene glycol, dipropylene glycol, polyalkylene glycols, such as triethylene glycol, polyethylene glycol, tripropylene glycol and polypropylene glycol; low alkyl ethers of multivalent alcohols, such as ethylene glycol monomethyl ether, ethylene glycol monoethyl ether, ethylene glycol monopropyl ether or ethylene glycol monobutyl ether, diethylene glycol monomethyl ether or diethylene glycol mono ethyl ether, triethylene glycol monomethyl ether or triethylene glycol mono ethyl ether; ketones and keto alcohols, especially such with 3 to 7 carbon atoms in their molecules, such as acetone, methyl ethyl ketone, diethyl ketone, methyl isobutyl ketone, methyl phenyl ketone, cyclopentanone, cyclo hexanone and diacetone alcohol; ethers such as dibutyl ether, tetrahydrofurane, dioxane or diisopropyl ether; esters such as ethyl formate, methyl formate, methyl acetate, ethyl acetate, propyl acetate, butyl acetate, phenyl acetate, ethylene glycol monoethyl ether acetate or acetic acid hydroxy ethyl ester; amids such as dimethyl formamide, dimethyl acetamide or N-methyl-pyrrolidone; as well as urea, tetramethyl urea and thiodiglycol; moreover, wetting agents or emulsifyers from the group of anionic, cationic, non-ionogenic, amphoteric or zwitterionic surface-active substances, such as fatty aclohol sulfates, alkyl-sulfonates, alkylbenzene sulfonates, alkyltrimethyl ammonium salts, alkylbetaines, α-olefin sulfonates, oxethylated fatty alcohols, oxethylated nonylphenoles, fatty acid alkanolamines, oxethylated fatty acid esters, fatty alcohol polyglycol ether sulfates, alkylpolyglucosides; thickening agents, such as high fatty alcohols, starch, cellulose derivatives, vaseline, paraffin oil, fatty acids and other fatty components in emulsified form, water-soluble polymeric thickening agents, for instance natural rubbers, guar gum, xanthan gum, carob flour, pectine, dextrane, agar-agar, amylose, amylopectine, dextrine, clays or synthetic hydrocolloides, such as polyvinyl alcohol; also conditioning agents, such as lanolin derivatives, cholesterol, pantothenic acid, water-soluble cationic polymers, protein derivatives, provitamins, vitamins, plant extracts, sugar and betaine; auxiliary agents, such as electrolytes, antioxidants, fatty amides, sequestrants, film-forming agents and preservatives.

The aforementioned compounds are used in the amounts usual for such purposes, for example, wetting agents and emulsifying agents in concentrations of about 0.5 to 30 % by weight, thickening agents of about 0.1 to 25 % by weight, conditioning agents in concentrations of about 0.1 to 5.0 % by weight.

The oxidizing agent and the alkalizer of the bleaching agent according to the present invention normally must be kept separately during storage and will be mixed direct prior to use. The agents according to the present invention may be packed in different ways. For instance the agents are in the form of a multi-component-kit, preferably a 2-component-kit or
3-component-kit, consisting of a component (A) containing the oxidizing agent and (B) containing at least one bleach booster of formula (I), at least one direct dye and if needed an agent for adjusting the pH. In another preferred embodiment the
2-component-kit consists of a component (A) containing at least one bleach booster of formula (I) and at least one oxidizing agent and a component (B) containing at least one direct dye and if needed an agent for adjusting the pH.

The pH of the ready-to-use agent is preferably from about 2 to about 12, more preferably from about 4 to about 11, and most preferably from about 6 to about 10,5.

The components are mixed prior to the application and the ready-to-use agent is spread on the fibers to be colored, if needed by addition of water or an aqueous solution containing usual cosmetic additives. The mixture is left on the fibers, such as hairs, at about 10 to 70 °C, preferably at about 20 to 50 °C, for about 5 to 60 minutes, preferably for about 30 and 50 minutes, then the fibers are rinsed with water, optionally washed with a shampoo and/or a conditioner, rinsed again with water and if needed dried.

The bleaching method according to the present invention achieves (especially on human hair), irrespective of different hair structures, uniform and highly reproducible bleaching results and especially natural and less orange tones in lighter shades.

Another object of the present invention is the use of the abovementioned type-2 bleach-stable direct dyes alone or in combination with at least one bleach booster according to the general formula (I) and at least one oxidant for acquiring a natural looking bleaching of keratin fibers, particularly humain hair, without undesired undertones.

The following examples further illustrate the invention in more detail, but they should not be construed as limiting the appended claims.

### Examples

### Example 1: Bleaching Agent (3-Component-Kit)

### Component A:

| | |
|---|---|
| ethanol | 10.000 g |
| laureth-4 | 1.000 g |
| methyl hydroxyethylcellulose | 0.800 g |
| sodium cyclohexanesulfamate | 25.000 g |
| Polyquaternium-7 (8 % in water) | 3.000 g |
| hydrogen peroxide (50 % aqueous solution) | 24.000 g |

### Component B:

| | |
|---|---|
| ethanol | 10.000 g |
| laureth-4 | 1.000 g |
| methyl hydroxyethylcellulose | 0.800 g |
| ammonium carbonate | 2.000 g |
| sodium cyclohexanesulfamate | 25.000 g |
| Polyquaternium-7 (8 % in water) | 3.000 g |
| ammonia (25 % aqueous solution) | 12.000 g |
| Acid Red No. 92 | 0.004 g |
| 3',3",4,5,5',5",6,7-octabromophenolsulfonphtalein | 0.012 g |
| water, demineralized | to 100.000 g |

### Component C:

| | |
|---|---|
| sodium persulfate | 100.000 g |

Prior to use 4.875 g of component A were mixed with 4.875 g of component B and 0.25 g of component C. The ready-to-use agent was applied to a medium brown natural hair strand and uniformly distributed with a brush. After an exposure time of 30 minutes at 40 °C, the hair was washed with a commercial shampoo, rinsed with lukewarm water, treated with a commercial conditioner, rinsed again with water and then dried. The hair showed a silver blond color.

### Example 2: Agent for Simultaneously Lightening and Toning Hair (3-Component-Kit)

### Component A:

| | |
|---|---|
| ethanol | 10.000 g |
| laureth-4 | 1.000 g |
| methyl hydroxyethylcellulose | 1.000 g |
| sodium cyclohexanesulfamate | 25.000 g |
| Polyquaternium-10 | 1.000 g |
| hydrogen peroxide (50 % aqueous solution) | 24.000 g |
| water, demineralized | to 100.000 g |

### Component B:

| | |
|---|---|
| ethanol | 10,000 g |
| laureth-4 | 1,000 g |
| methyl hydroxyethylcellulose | 1.000 g |
| sodium cyclohexanesulfamate | 25.000 g |
| Polyquaternium-10 | 1.000 g |
| ammonia (25 % aqueous solution) | 12.000 g |
| Acid Red No. 92 | 0.006 g |
| 3',3",4,5,5',5",6,7-octabromophenolsulfonphtalein | 0.014 g |
| water, demineralized | to 100.000 g |

### Component C:

| | |
|---|---|
| sodium persulfate | 100.000 g |

Prior to use 4.85 g of component A were mixed with 4.85 g of component B and 0.3 g of component C. The ready-to-use agent was applied to a medium brown natural hair strand and uniformly distributed with a brush.
After an exposure time of 30 minutes at 40 °C, the hair was washed with a commercial shampoo, rinsed with lukewarm water, treated with a commercial conditioner, rinsed again with water and then dried. The hair showed an ash blond color.

Unless otherwise indicated, all percentages in the present patent application are percentages by weight.

## Claims

1. A process for bleaching keratin fibers which is **characterized by** applying to the fiber a ready-to-use agent having a basic pH and containing
a) at least one bleach booster according to the general formula **(I)** 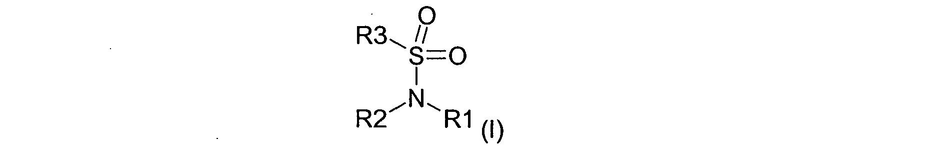 wherein **R1** is a hydrogen, **R2** is hydrogen, a substituted or unsubstituted C1- to C12-alkyl group, a substituted or unsubstituted C1- to C12-monohydroxy-alkyl group, a substituted or unsubstituted C2- to C12-polyhydroxy alkyl group, or a substituted saturated, unsaturated or aromatic 4- to 8-membered carbocycle or heterocycle, and **R3** is a OR-Group, with **R** being equal to hydrogen, an ammonium group or an alkali metal or an earth alkali metal atom; and
b) at least one appropriate type-2 bleach-stable direct dye and
c) at least one oxidant; and
after an exposure time of 5 to 60 minutes at a temperature of 10 to 70 °C rinsing the fiber with water.

2. Process according to claim 1, **characterized in that** the bleach booster of the general formula (I) is selected from the group consisting of sodium cyclohexanesulfamate; ammonium cyclohexanesulfamate;
potassium cyclohexanesulfamate; cyclohexane sulfamic acid;
sulfamic acid; sodium sulfamate; potassium sulfamate;
ammonium sulfamate; sodium phenylsulfamate;
ammonium phenylsulfamate; potassium phenylsulfamate;
phenyl sulfamic acid; sodium benzylsulfamate;
ammonium benzylsulfamate; potassium benzylsulfamate;
benzyl sulfamic acid; sodium isopropylsulfamate;
ammonium isopropylsulfamate; potassium isopropylsulfamate;
isopropyl sulfamic acid; sodium methylsulfamate;
ammonium methylsulfamate; potassium methylsulfamate;
methyl sulfamic acid; sodium t-butylsulfamate;
ammonium t-butylsulfamate; potassium t-butylsulfamate and
t-butyl sulfamic acid.

3. Process according to claim 1 or 2, **characterized in that** the bleach booster is contained in the ready-to-use agent in an amount from 0.01 to 70 weight percent.

4. Process according to one of claims 1 to 3, **characterized in that** the type-2 bleach-stable direct dye is selected from the group consisting of 3-(2',6'-diaminopyridyl-3'-azo)pyridine, N,N-di(2-hydroxyethyl)-3-methyl-4-((4-nitrophenyl)azo)-aniline, 3-diethylamino-7-(4-dimethylaminophenylazo)-5-phenylphenazinium chloride, 4-(2-thiazolylazo)-resorcinol, 4-((4-phenylamino)azo)benzosulfonic acid sodium salt, 1-((3-aminopropyl)-amino)-9,10-anthracenedione, 3',3",4,5,5',5",6,7-octabromphenol-sulfonphtalein, 1-((4-amino-3,5-dimethylphenyl)(2,6-dichlorophenyl)-methylene)-3,5-dimethyl-4-imino-2,5-cyclohexadiene-phosphoric acid (1:1), 3',3",5',5"-tetrabromo-m-cresol sulfonephthalein, 2,4-dinitro-1-naphthol-7-sulfonic acid disodium salt, 4-[2'-hydroxy-1'-naphthyl)azo]benzosulfonic acid sodium salt, 3',6'-dihydroxy-2',4',5',7'-tetraiodospiro-[isobenzofuran-1(3H), 9'-(9H)xanthen]-3-one disodium salt, 6-hydroxy-5-((2-methoxy-5-methyl-4-sulfophenyl)azo)-2-naphthalenesulfonic acid disodium salt, 2,4-dinitro-1-naphthol sodium salt, 2',4',5',7'-tetrabromo-4,5,6,7-tetrachloro-3',6'-dihydroxyspiro-(isobenzofuran-1(3H), 9'-[9H]xanthen]-3-one disodium salt, 4-(2-hydroxy-1-naphthylazo)-3-methylbenzenesulfonic acid sodium salt, 2-amino-1,4-naphthalenedione, dithizone (1,5-diphenylthiocarbazone), N-((2-hydroxyethyl)-2-nitro-4-trifluoromethyl)aniline, N-(2-hydroxyethyl)-4-nitroaniline and 4-chloro-N-(2,3-dihydroxypropyl)-2-nitroaniline.

5. Process according to one of claims 1 to 4, **characterized in that** the type-2 bleach-stable direct dye is contained in the ready-to-use agent in a total amount from 0.0001 to 10 weight percent.

6. Process according to one of claims 1 to 5, **characterized in that** the oxidizing agent is selected from the group consisting of hydrogen peroxide, inorganic alkali metal peroxides, organic peroxides, inorganic perhydrate salt bleaching compounds, monohydrates or tetrahydrates of inorganic perhydrate salt bleaching compounds, alkyl peroxides, aryl peroxides, peroxidases and mixtures of these compounds.

7. Process according to claim 6, **characterized in that** the oxidizing agent is selected from the group consisting of hydrogen peroxide, percarbonates, persulfates and combinations thereof.

8. Process according to one of claims 1 to 7, **characterized in that** the oxidizing agent is contained in a total amount of from 0.1 to 15 weight percent.

9. Process according to one of claims 1 to 8, **characterized in that** the ready-to-use agent has a pH of from 2 to 12.

10. Process according to one of claims 1 to 9, **characterized in that** after rinsing with water the fiber is washed with a shampoo and/or a conditioner and rinsed again with water.

11. Process according to one of claims 1 to 10, **characterized in that** finally the keratin fiber is dried.

12. Use of type-2 bleach-stable direct dyes selected from the group consisting of 3-(2',6'-diaminopyridyl-3'-azo)pyridine, N,N-di(2-hydroxyethyl)-3-methyl-4-((4-nitrophenyl)azo)-aniline, 3-diethylamino-7-(4-dimethylamino-phenylazo)-5-phenylphenazinium chloride, 4-(2-thiazolylazo)-resorcinol, 4-((4-phenylamino)azo)benzosulfonic acid sodium salt, 1-((3-aminopropyl)-amino)-9,10-anthracenedione, 3',3",4,5,5',5",6,7-octabromphenol-sulfonphtalein, 1-((4-amino-3,5-dimethylphenyl)(2,6-dichlorophenyl)-methylene)-3,5-dimethyl-4-imino-2,5-cyclohexadiene-phosphoric acid (1:1), 3',3",5',5"-tetrabromo-m-cresol sulfonephthalein, 2,4-dinitro-1-naphthol-7-sulfonic acid disodium salt, 4-[2'-hydroxy-1'-naphthyl)azo]benzosulfonic acid sodium salt, 3',6'-dihydroxy-2',4',5',7'-tetraiodospiro-[isobenzofuran-1 (3H), 9'-(9H)xanthen]-3-one disodium salt, 6-hydroxy-5-((2-methoxy-5-methyl-4-sulfophenyl)azo)-2-naphthalene-sulfonic acid disodium salt, 2,4-dinitro-1-naphthol sodium salt, 2',4',5',7'-tetrabromo-4,5,6,7-tetrachloro-3',6'-dihydroxyspiro-(isobenzofuran-1 (3H), 9'-[9H]xanthen]-3-one disodium salt, 4-(2-hydroxy-1-naphthylazo)-3-methylbenzenesulfonic acid sodium salt, 2-amino-1,4-naphthalenedione, dithizone (1,5-diphenylthiocarbazone), N-((2-hydroxyethyl)-2-nitro-4-trifluoromethyl)aniline, N-(2-hydroxyethyl)-4-nitroaniline and 4-chloro-N-(2,3-dihydroxypropyl)-2-nitroaniline, in type-2 bleaches for acquiring a natural looking bleaching of keratin fibers without undesired undertones.

13. Use of a combination of a) at least one bleach booster according to the general formula (I) 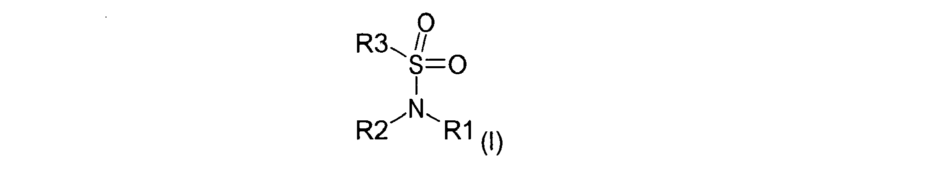 wherein **R1** is a hydrogen, **R2** is hydrogen, a substituted or unsubstituted C1- to C12-alkyl group, a substituted or unsubstituted C1- to C12-monohydroxy-alkyl group, a substituted or unsubstituted C2- to C12-polyhydroxy alkyl group, or a substituted saturated, unsaturated or aromatic 4- to 8-membered carbocycle or heterocycle, and **R3** is a OR-Group, with **R** being equal to hydrogen, an ammonium group or an alkali metal or an earth alkali metal atom; and
b) at least one appropriate type-2 bleach-stable direct dye and
c) at least one oxidant;
for acquiring a natural looking bleaching of keratin fibers without undesired undertones.

14. Use according to claim 13, **characterized in that** the type-2 bleach-stable direct dye is selected from the group consisting of 3-(2',6'-diaminopyridyl-3'-azo)pyridine, N,N-di(2-hydroxyethyl)-3-methyl-4-((4-nitrophenyl)azo)-aniline, 3-diethylamino-7-(4-dimethylamino-phenylazo)-5-phenylphenazinium chloride, 4-(2-thiazolylazo)-resorcinol, 4-((4-phenylamino)azo)benzosulfonic acid sodium salt, 1-((3-aminopropyl)-amino)-9,10-anthracenedione, 3',3",4,5,5',5",6,7-octabromophenol-sulfonphtalein, 1-((4-amino-3,5-dimethylphenyl)(2,6-dichlorophenyl)-methylene)-3,5-dimethyl-4-imino-2,5-cyclohexadiene-phosphoric acid (1:1), 3',3",5',5"-tetrabromo-m-cresol sulfonephthalein, 2,4-dinitro-1-naphthol-7-sulfonic acid disodium salt, 4-[2'-hydroxy-1'-naphthyl)azo]benzosulfonic acid sodium salt, 3',6'-dihydroxy-2',4',5',7'-tetraiodospiro-[isobenzofuran-1 (3H), 9'-(9H)xanthen]-3-one disodium salt, 6-hydroxy-5-((2-methoxy-5-methyl-4-sulfophenyl)azo)-2-naphthalene-sulfonic acid disodium salt, 2,4-dinitro-1-naphthol sodium salt, 2',4',5',7'-tetrabromo-4,5,6,7-tetrachloro-3',6'-dihydroxyspiro-(isobenzofuran-1(3H), 9'-[9H]xanthen]-3-one disodium salt, 4-(2-hydroxy-1-naphthylazo)-3-methylbenzenesulfonic acid sodium salt, 2-amino-1,4-naphthalenedione, dithizone (1,5-diphenylthiocarbazone), N-((2-hydroxyethyl)-2-nitro-4-trifluoromethyl)aniline, N-(2-hydroxyethyl)-4-nitroaniline and 4-chloro-N-(2,3-dihydroxypropyl)-2-nitroaniline.
